# EUROPEAN PATENT SPECIFICATION

(11) **EP 2 375 884 B1**
(45) Date of publication and mention of the grant of the patent: **18.05.2016**
(21) Application number: 09836439.1
(22) Date of filing: 23.12.2009
(51) Int. Cl.: A01G 33/00, A01H 13/00

(54) **CARRIER FOR GROWING MACROALGAE IN A WATER VOLUME, AND AN ARRANGEMENT FOR SUSPENDING SUCH CARRIERS**
TRÄGER ZUR ZÜCHTUNG VON MAKROALGEN IN EINEM WASSERVOLUMEN UND ANORDNUNG ZUR AUFHÄNGUNG DERARTIGER TRÄGER
SUPPORT POUR LA CROISSANCE DE MACRO-ALGUES DANS UN VOLUME D'EAU, ET AGENCEMENT POUR LA SUSPENSION DE CES SUPPORTS

(30) Priority: 30.12.2008 NO 20085410
(43) Date of publication of application: 19.10.2011
(73) Proprietor: Seaweed Energy Solutions AS, 7018 Trondheim (NO)
(72) Inventor: BAKKEN, Paal Andreas, 2750-535 Cascais (PT)
(74) Representative: Acapo AS
(86) International application number: PCT/NO2009/000444
(87) International publication number: WO 2010/077146

(56) References cited:
- WO-A1-2005/082129
- GB-A- 2 051 548
- JP-A- 9 172 903
- JP-A- 11 308 904
- JP-A- 2003 047 364
- BELA HIERONYMUS BUCK ET AL: "The offshore-ring: A new system design for the open ocean aquaculture of macroalgae", JOURNAL OF APPLIED PHYCOLOGY, KLUWER ACADEMIC PUBLISHERS, DO, vol. 16, no. 5, 1 October 2004 (2004-10-01), pages 355-368, XP019247746, ISSN: 1573-5176

## Description

The invention relates to a sea bottom moored carrier arrangement for automated seeding, farming, and harvesting of macroalgae in the sea as described in the introductory part of claim 1, a use of the arrangement, and a method for growing of seaweed.

### Background

There are two categories of algae; macroalgae and microalgae. This invention relates to the farming of macroalgae commonly known as seaweed. Farming of seaweeds has a long tradition in Asia. Where seaweed is cultivated for production of food ingredients, feedstock, carrageenan, agar, alginates and medical applications. Recently there is a growing interest to use seaweed for energy, such as biogas, bioethanol, electricity etc., pharmaceuticals, petrochemical replacements, and production of bioplastics.

Currently, seaweeds are seeded on ropes which are attached manually to larger ropes in the sea. The ropes are anchored with multiple points and there are various ways of deployment in the sea, vertically as well as horizontally.

There is a need for a more industrial, automated seeding, farming and harvesting system for seaweeds, particularly for being competitive as a energy crop.

From Japanese patent application 2006325563 it is known to suspend multiple sheet like carriers hanging in ropes from a series of floating bodies. This arrangement suffers from various drawbacks, including the need for manual work for mounting and harvesting of the carriers, and the sensitivity of the carriers in a water mass with currents.

From Japanese patent 3083529 it is known to provide a holding tool for growing seaweed, comprising frames with a net, to which seed ropes are attached. The ropes have spores adhered thereto and a resin coated fertilizer being provided in a container. This system demands manual work in sowing the spores and harvesting the algae, which will not allow large scale growing for energy purposes.

From Chinese patent 1132275 it is known to prepare a base for growing seaweed by using a polypropylene web as a raw material. From Japanese patent 9000096 it is known to prepare an algae culturing net from polyvinyl. From Japanese patent 11262337 it is known a net for culturing seaweed which improves the yield, by having the net prepared without knots. Neither of these nets have been suitable for large scale, mechanically operated growing of seaweed.
It is also known from Japanese patent application to suspend a net of considerable length with inclined ropes to anchors.
JP 11 308904 A discloses a seeding sheet with 2-50% holes to be placed on the sea bottom for establishment of macroalgae on the sea bottom.
A sea bottom moored ring-structured arrangement for cultivation of macroalgae by means of cultivation lines is suggested for exposed open sea aquaculture purposes (WO 2005/082129; Buck, B. H. & Buchholz, C. M. 2004. The offshore-ring: A new system design for the open ocean aquaculture of macroalgae. J. Appl. Phycol. 16: 355-368).

From GB 2051548 A a structure is known for growing of seaweed comprising a heat resistant sheet substrate which can be placed in a drying oven to yield dried seaweed.
Thus, there is no carrier proposed which is available for both mechanically handling and mechanically harvesting of the grown seaweed. Furthermore, there is no carrier proposed that ensures no entanglement of marine mammals, such as whales.
Furthermore, seaweed farms are currently located in protected or semi-protected areas of the ocean since they are not constructed to withstand the extreme forces in the open ocean environment

### Objects of the invention

The main object of the invention is to make the whole seaweed cultivation process, including seeding, farming, maintenance, transport and harvesting, more efficient, including providing a structure suitable for automated farming, allowing the use of machines for applying seeds and for harvesting.

A further object is to provide a carrier which can stand flow and movement of water in an open sea area, allowing seaweed culture in larger areas of the sea.

It is a further object to provide a carrier which prohibits mammal entanglement, which is a problem with rope and net based seaweed farming systems.

Further objects will be disclosed in the following description of the invention.

### The Invention

A sea bottom moored carrier arrangement for automated seeding, farming, and harvesting of macroalgae in the sea, comprising one or more carriers suspended in the water, suitable to withstand changing flow directions, and means for attachment of the carrier to the mooring according to the invention is described in claim 1. Each carrier comprises a sheet of a flexible material selected from the group consisting of a solid, a laminated, and a reinforced material, where the surface of the sheet is configured to retain seeds and spores, the sheet having apertures which allow water flow to pass from one side of the sheet to the other, thereby reducing the resistance to waves and currents and enabling a flow of nutrients in the water to circulate through the carrier, wherein each carrier is floating in the water by means of its positive buoyancy, and/or by being provided with buoyant means, and the carrier being connected to the means for attachment, at one point or edge of the sheet.

Thus, the carrier comprises a sheet of a flexible material which has a pattern of apertures allowing water to pass from one side of the carrier to the other. The carrier can comprise a web of ribbons, but consists preferably of a sheet with apertures in a pattern. The carrier is designed to grow large wild brown seaweeds or similar macroalgae.

Preferred embodiments of the arrangement according to the present invention are also described in the dependent claims 2-13.

Claim 14 relates to a use of a sea bottom moored carrier arrangement according to the invention for automated seeding, farming and harvesting of seaweed wherein said arrangement comprises one or more carriers suspended in the water, suitable to withstand changing flow directions, and means for attachment of the carrier to the mooring. Each carrier comprises a sheet of a flexible material selected from the group consisting of a solid, a laminated, and a reinforced material, where the surface of the sheet is configured to retain seeds and spores, the sheet having apertures which allow water flow to pass from one side of the sheet to the other, thereby reducing the resistance to waves and currents and enabling a flow of nutrients in the water to circulate through the carrier, wherein each carrier is floating in the water by means of its positive buoyancy, and/or by being provided with buoyant means, and the carrier being connected to the means for attachment, at one point or edge of the sheet.

Further preferred embodiments related to the use of the invention are described in the claims 15 and 16.

Claim 17 relates to another aspect of the present invention being a method for growing of sea weed. The method comprises the following steps:
(i) seeding of the seeds or spores on at least one carrier for growing of seaweed, wherein said carrier comprises a sheet of a flexible material selected from the group consisting of a solid, a laminated, and a reinforced material, where the surface of the sheet is configured to retain seeds and spores deposited thereon,
   - the sheet having apertures which allow water flow to pass from one side of the sheet to the other, thereby reducing the resistance to waves and currents and enabling a flow of nutrients in the water to circulate through the carrier,
   - wherein each carrier is floating in the water by means of its positive buoyancy, and/or by being provided with buoyant means, and
(ii) suspending and arranging of said carrier in the sea by attachment to sea bottom moored means for attachment, at one point or edge of the carrier sheet. Preferred methods according to the invention are disclosed in dependent claims 18-20.

The sheets may be made of a solid or porous material, or may be laminated from two or more sub-sheets or assembled from fibres. The material may be organic or any other material providing flexibility, which preferably is biodegradable. The carrier sheet may be manufactured for one-time use, to be recycled mechanically at harvesting, preferably with mechanical means, or being reusable several times.

The carrier sheet may have a negative or positive buoyancy, the buoyancy being adjustable with additional means, such as ropes or other elements with positive or negative buoyancy.

The carrier may have a rectangular or similar form, including various leaf forms. It may also be in the form of a long blanket, attached to a mooring means at one or both ends.

The carrier may be designed for attachment at one or more points or sides to other carriers and/or to an attachment means, like a rope. Alternatively, the carriers may have a single point attachment at one corner.

Generally, the carrier according to the invention can be moored in one of following ways:
- Single point mooring to the sea bottom
- Single point mooring to horizontal lines
- Single point mooring to vertical lines
- Multiple point mooring
- Edgewise mooring to a line
- Edgewise mooring to a similar carrier

The carrier may also be arranged in groups of two or more in various arrangements, as will be shown in the Examples.

The material of the carrier sheets may be translucent or opaque, depending on the way of utilizing. When used in several stacks in a water volume, a translucent material will be preferable, to gain more light for the photosynthesis.

The top layer or both the top and bottom layer may have a hoarse surface for carrying spores or seed to be placed on the carrier, e.g. by spraying or immersing into a suspension.

The carrier according to the invention may be arranged for use in an unidirectional flow of water. It may also be suspended or moored to stand multidirectional flows.

The apertures central to this invention may be provided by punching or as a part of the process generating the sheet material of the carrier. The apertures may be longitudinal slots, or other kinds of openings arranged in a pattern suitable for the area of use and the sea area in which it is used.

The carrier sheet is preferably homogeneous, but may have reinforcement, e. g. In the form of an integrated net. It may also have a rim, e.g. along all sides, which is enlarged to stand the use of attachment means or which may be provided with a rope suitable for attachment to suspension elements.

When providing a carrier in the form of a long carpet, the buoyancy properties may vary along the length to provide an undulated position in the water. This may make the carrier withstand higher waves and allow a higher growth area.

An important consideration in the design of the carrier is the challenges created by the size and by the need for mechanical handling. Mechanical handling poses restrictions as to the width of a sheet being manufactured e.g. in a factory site located close to the sea, for expelling into the sea. The carrier sheets thus should be suitable for linking together multiple longitudinal webs. The elements of this linking should be suitable for cutting or disconnecting with mechanical means at harvesting. The connection between carrier elements in the operative mode should allow both for heavy waves and for whales and other mammals posing a risk to prior art seaweed farms.
The carrier according to the invention may be suspended like a carpet in a single horizontal layer or with multiple horizontal layers in a stack, e. g. 2 -3 layers, with a multiple point suspension. Alternatively single carriers or groups of carriers may be arranged with a single point mooring. This is particularly suitable when the seaweed culture is placed in an area with changing directions of flow of the water and/or in areas with storms, posing a risk to large structures of carriers with suspension on all sides.
The carriers according to the invention may be manufactured of a material which incorporates a growth substance, normally in the form of a fertilizer for the particular seaweeds. Alternatively or additionally, growth substances may be added as a deposit on the carrier sheet.
The growth substance can be various organic or part organic waste, e.g. residues from handling of municipal waste water, or from various organic materials. Included is also the recycling of the carrier according to the invention, when this carrier is of organic material.
The carrier according to the invention may be dragged into the sea, but may also be delivered in rolls, for seagoing transport to a site remote from the manufacturing site.
The features of the invention are described in the claims, which should be interpreted to cover the details of the description.
The invention can be used for growing seaweed for various purposes. It is however mainly intended for high volume growing, for purposes such as producing bioethanol, biogas and various co- and byproducts.

### Examples

Embodiments of the invention and comparative examples are described in the following, with reference to the drawings, in which
Figure 1 is showing a perspective view of an embodiment of a carrier according to the invention,
Figure 2 and 3 are showing a perspective view of two embodiments for suspending a rectangular carrier according to the invention in a water volume,
Figure 4 is showing in a comparative example a perspective view of two rectangular carrier chained together,
Figure 5 is showing a perspective view of a group of rectangular carrier with single point mooring on poles extending from the sea bottom according to the present invention,
Figure 6 is showing in a comparative example a perspective view of a circular or ellipsoid carrier with a single point mooring,
Figure 7 is showing a further embodiment of the present invention being a perspective view of a leaf shaped carrier with single point mooring,
Figures 8 - 10 are showing various group structures of carriers according to the present invention, all being single point moored,
Figure 11 is showing a side view of a further embodiment of the invention, while
Figure 12 is showing a perspective view of a still further embodiment of the invention, with a platform for carrying growth carriers or being a growth carrier.

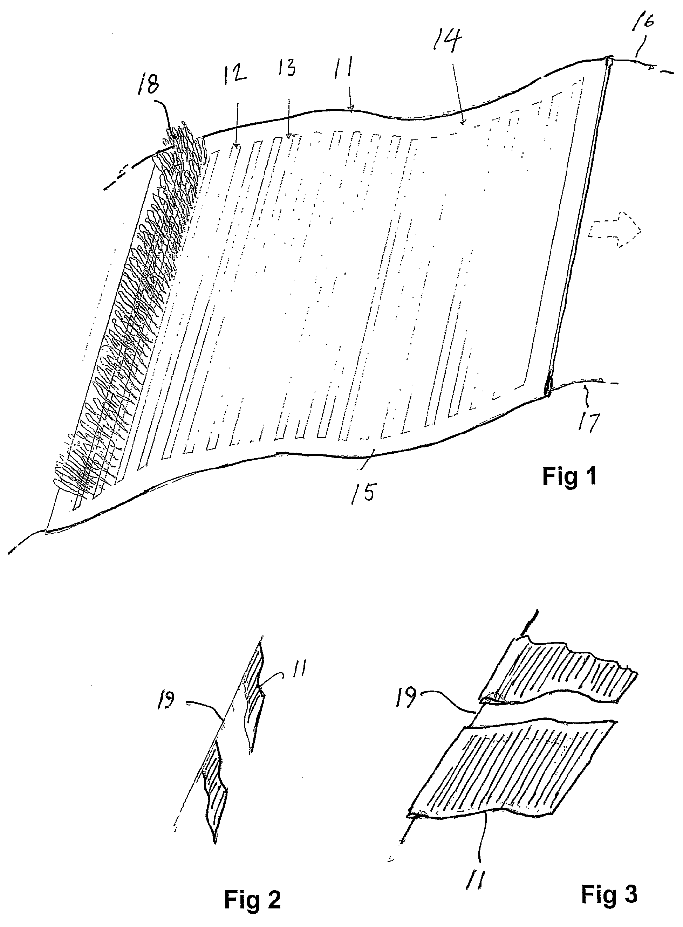

Figure 1 shows a carrier 11 prepared in the form of a rectangular blanket which is flexible at least longitudinally, i. e. on crosswise extending axis's. The carrier 11 is provided with series of crosswise extending slot 12 leaving intermediate narrow segments 13 of the base material. The carrier 11 can be manufactures of a continuous web, the slots 12 being cut or punched in an integrated or separate process.

On the longitudinal sides of the carrier, a continuous rim 14, 15 is left on both sides. The purpose of the rims 14, 15 is to enable stability and handling of the longitudinal carrier 11 on application of spores, on suspending in a water volume or when harvesting the algae with mechanical means.

The rims 14, 15 of the carrier 11 can be provided with an embedded line or rope 16, 17, partly for reinforcing, partly for suspending, partly for handling.

The ends of the carrier 11 can have an extended solid portion available for attachment means, as illustrated in Figure 1, for a attachment line as shown in Figure 3, or for a reinforcement rod.

The carrier can be manufactured by any sheet or web of plastic, textile or combination of such material, which has the property of holding spores or seeds in a growth period in water and which has the mechanical stability and endurance to stand repeated mechanical handling and mechanical harvesting of the growth product.

The carriers may be manufactured of any plastic or organic material or combination of material which is compatible with the object of holding a large number for spores or seeds for growing, and which can endure being suspending continuously in the water.

The carriers can have buoyancy for staying horizontal or for hanging down. They may have a rugged or porous surface, or even be covered by a layer of a different growing substrate, which may have a fertilizer.

The apertures 12 will allow water flow from one side of the carrier to the opposite, reducing the resistance to waves and tidal currents as well as enabling a flow of nutrients in the water to circulate through the carrier.

The seeds or spores can be applied to the carrier by several methods, including spraying with nozzles, immersing in a bath, rolling with a fluid suspension, strewing or even by electrostatical means. The object is to have this process implemented by mechanical means, to reduce the manual efforts and increase the speed.

The applying of the spores may be done on shore, in an inlet (pool), on a vessel or on a platform arranged for serving a macroalgae culture or seaweed farming plant.

Figure 1 also illustrated the growing of seaweeds 18 on a part of the carrier 11.

This design of the carrier 11 will allow mechanical handling and harvesting of the seaweeds, by exposing the active side or active sides of the carrier which enables more flexibility to move the carrier, e.g. by the use of a suction head, and to remove the seaweed from the carrier.

The rectangular form of the carrier shown in Figure 1 is only one option. The carrier may have any shape which is suitable for mooring and handling under the given conditions regarding water flow, wave height, and other growth conditions.

The structure of the carrier can also be that of a woven carpet, the basic material be of plastic or grown yarn, or even combination. The requirement is that the material has a general uniform pattern of apertures, like the slots 12 shown or like holes or combinations. The carrier may even be prepared as a sheet like open structure of ropes providing growth areas for seaweed. As an option, a net of sufficient strength may be covered by a perforated layer of a medium suitable for growing seaweed. This medium may even be recycled in a harvesting process.

Figures 2 and 3 are showing various embodiments of suspending the carrier according to the invention. In Figure 2 a series of rectangular carriers 11 are suspended vertically from a line 19. In this example, the lower end of the carriers 11 may have a ballast line. In Figure 3 a series of carriers 11 are attached at one end or side to a line 19, the buoyancy of the carrier providing a generally horizontal state.

Both embodiments will allow the traffic of mammals without entanglement. In both embodiments, series of parallel lines 18 and 19 may be arranged to cover an area of the sea.

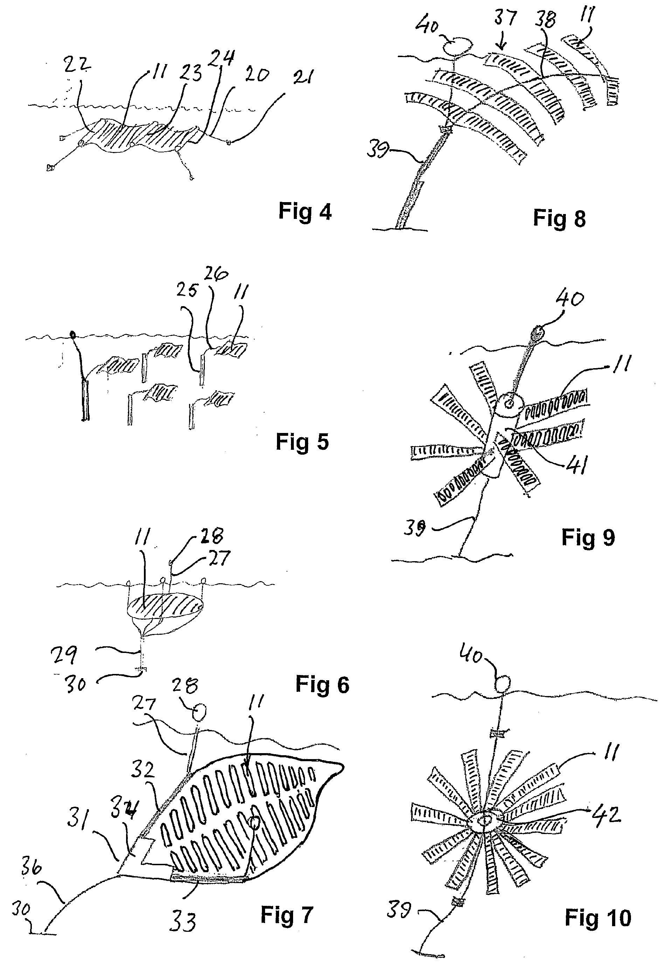

Figure 4 shows in a comparative example two rectangular carriers 11 which are chained together and moored at the resulting four corners with lines 20 from anchors 21 at the sea bottom. At the ends of the carriers 11, flexible tubes 22, 23, 24 are integrated.

Figure 5 shows a group of five rectangular carrier 11, each being moored to a vertical stem 25, rising from the sea bottom. Each carrier 11 is connected with a line 26, allowing the carriers 11 to adjust horizontally or inclined to the flow of water.

Figure 6 shows a further comparative example where a circular or ellipsoid carrier 11 is suspended in the water with lines 27 from four buoyancy bodies 28 and with a central mooring line 29 connected to an anchor 30 at the sea bottom.

Figure 7 shows a single carrier 11 with a single point mooring corresponding to the embodiment of Figure 5. The carrier 11 has lower corner 31 with a pair of flexible rims 32, 33 joined in a corner element 34 attached to the mooring line 35. The corner element 34 may be any suitable reinforcement element attached to the flexible rims 32, 33, which may be of flexible tubes.

The outer end of each flexible rim 32, 33 is connected to a buoyancy element 28 with a line 27.

The shape of the carrier 11 may be like a natural leaf or oblong sheet, suited for suspension in a flowing water volume. The carrier might be reinforced in the middle and from the middle sideward similar to a natural leaf. The line 36 connecting the carrier 11 to the anchor equipment is preferably a flexible rod which will hold the carrier in an oblique position in the water.

Carriers with single point mooring are suitable for offshore / open ocean deployment since the carriers will be pushed down or submerged further by the natural forces of the high waves.

Figures 8 - 10 are showing three different group structures of carriers 11, all having a single point mooring.

Figure 8 shows a multi foiled leaf 37 with a central flexible stem 38 connected to a stem 39.

Figure 9 shows a series of ribbon shaped carriers 11 attached to a cylindrical core 41 and pointing in different directions from different heights.

Figure 10 shows a series of carriers 11 in a daisy structure on a central ring 42.

Common to these embodiments: A buoyancy body 40 is connected to the stems 39, partly to show the site of this group of carriers, partly to keep the stem 39 upright.

Common to all single point mooring embodiments is the arrangement in a sufficient distance to prohibit entanglement of neighboring carriers during storms.

The advantage of this suspension is its unidirectional properties and its insensitiveness for whales. It will also be possible to combine this suspension of the carriers with mechanical handling and harvesting.

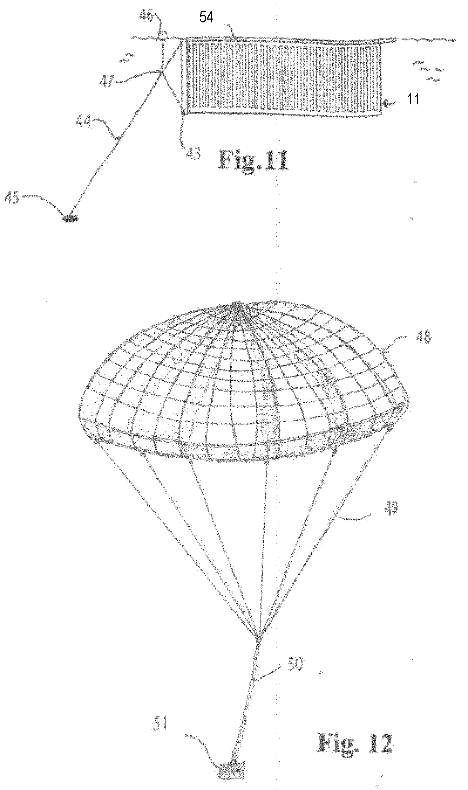

Figure 11 is showing a rectangular carrier 11 with a flexible rod or line 54, e.g. a plastic hose, attached at an upper long side and a rigid bar 43 attached at an adjoining short side. The structure of the carrier 11 and the attached part is chosen to maintain the carrier 11 vertically in the sea. To maintain the carrier 11 on a determined location, a line 44 is extending between a bottom weight 45 and a buoy 46 at the surface. A craw foot 47 is attaching the carrier 11 to the line 44.

Figure 12 is showing a still further embodiment of the invention. A circular element 48 of a flexible web, e.g. net as shown in the drawing, or a plastic foil or a fabric, which may be closed or having openings, is suspended in the sea, to have the form of a parachute, a mushroom or an umbrella. The circular element 48 may have its own buoyancy or being provided with buoyancy elements. It is even conceivable to provide freshwater inside a close top of a circular element, to provide buoyancy.

To maintain its form and position, the rim of the circular element 48 is tied in by a series of lines 49 connected like the lines of a parachute to a support line 50 extending to a weight 51 suspended in the sea or resting on the seabed. The series of lines 49 may be replaced or reinforced by a conical net (not shown).

The circular element 48 may be used in various ways. It may have the qualities of a carrier for growing seaweed on the element itself. The supply of seeds may even be done with the element suspended in the sea. This may be achieved from a boat with a suitable dispenser for seeds in a fluid.

The circular element 48 as a single structure or as a part of an assembly may be suspended in an adjustable way in the sea, to control the submersion to a suitable depth. For this purpose, the line 50 may be connected to a buoy. This will allow to select the most suitable growth conditions for each part of the growth period and for each particular environment.

The harvesting may be done from a boat, with equipment acting direct on the circular element 48. In an alternative embodiment, the circular element 48 may act as a platform for suspending a carrier according to the invention. Then a carrier being provided with seeds may be disposed on the platform. After the growth period, this carrier with seaweed may be harvested.

Generally, there are three ways of providing seeds or spores to the carriers:
- by spraying or other application onshore
- by spraying offshore, i. e. in the sea
- by natural spore attachment.

## Claims

1. A sea bottom moored carrier arrangement for automated seeding, farming, and harvesting of macroalgae in the sea, comprising one or more carriers (11) suspended in the water, suitable to withstand changing flow directions, and means for attachment of the carrier to the mooring, **wherein**
- each carrier (11) comprising a sheet of a flexible material selected from the group consisting of a solid, a laminated, and a reinforced material, where the surface of the sheet (11) is configured to retain seeds and spores,
- the sheet having apertures (12) which allow water flow to pass from one side of the sheet to the other, thereby reducing the resistance to waves and currents and enabling a flow of nutrients in the water to circulate through the carrier,
- wherein each carrier (11) is floating in the water by means of its positive buoyancy, and/or by being provided with buoyant means (40,28), and
- the carrier (11) being connected to the means for attachment (19,38,39,41,42), at one point or edge of the sheet.

2. Arrangement according to claim 1, **wherein** the carrier or group of carriers is moored by single point mooring to the sea bed via a mooring line (26,36,39).

3. Arrangement according to any of the preceding claims, **wherein** the surface of the sheet is hoarse, rugged, or porous to retain seeds and spores.

4. Arrangement according to claim 1, **characterized in that** the apertures (12) are generally parallel slots or series of generally circular openings.

5. Arrangement according to one of the claims 1 to 4, **characterized in that** the carrier is rectangular, leaf shaped or oblong, with a rim (14,15) on at least on side for suspending.

6. Arrangement according to claim 5, **characterized in that** the rim (14,15) has an embedded line (16,17) for reinforcement and suspension.

7. Arrangement according to any of the preceding claims, **characterized in that** it comprises a laminated structure with a relatively strong and thin foil material and a layer of growth substance, which may be biodegradable.

8. Arrangement according to any of the claims 1 to 7, **characterized in that** the sheet includes a net of reinforcement lines.

9. Arrangement according to one of the claims 1 to 8, **characterized in that** it comprises a fertilizer and/or nutrient substance for enhancing the growth of the macroalgae.

10. Arrangement according to one of the claims 1 to 9, **characterized in that** the buoyancy is varying along the length of an extended carrier, to achieve an undulated shape when suspended in the water.

11. Arrangement according to claim 1, particularly with a carrier in rectangular form, **characterized in that** a flexible rod (42) with buoyancy is attached or integrated at one side of the carrier, and a more rigid rod (43) is attached at an adjoining side, said more rigid rod being provided for suspension of the carrier (41) in a generally vertical position, with the freedom to follow the flow of the sea.

12. Arrangement according to claim 1, **characterized in that** it comprises attachment means for suspending a carrier in form of a generally circular element (48) with buoyancy and with lines or a net or a foil or fabric connecting the edge in a parachute manner, said lines and/or net being connected to a weight (51), and where the circular element (48) is a platform and said carrier is disposed thereon.

13. Arrangement according to claim 1, **whereby** a group of carriers is attached to one means for attachment, such as a line (19), stem (38,39) or central ring (42).

14. Use of a sea bottom moored carrier arrangement for automated seeding, farming and harvesting of seaweed wherein said arrangement comprises one or more carriers (11) suspended in the water, suitable to withstand changing flow directions, and means for attachment of the carrier to the mooring, where
- each carrier (11) comprising a sheet of a flexible material selected from the group consisting of a solid, a laminated, and a reinforced material, where the surface of the sheet (11) is configured to retain seeds and spores,
- the sheet having apertures (12) which allow water flow to pass from one side of the sheet to the other, thereby reducing the resistance to waves and currents and enabling a flow of nutrients in the water to circulate through the carrier,
- wherein each carrier (11) is floating in the water by means of its positive buoyancy, and/or by being provided with buoyant means (40,28), and
- the carrier (11) being connected to the means for attachment (19,38,39,41,42), at one point or edge of the sheet.

15. Use according to claim 14, wherein the carrier is suspended vertically or horizontally in the water volume.

16. Use according to claim 14, wherein the carrier is manufactured of plastic, organic material or a combination, which can hold a large number of spores or seeds for growing and which can endure being suspended continuously in water.

17. Method for growing of seaweed **characterized in that** the method comprises the following steps:
(i) seeding of the seeds or spores on at least one carrier for growing of seaweed, wherein said carrier comprises a sheet of a flexible material selected from the group consisting of a solid, a laminated, and a reinforced material, where the surface of the sheet (11) is configured to retain seeds and spores deposited thereon,
- the sheet having apertures (12) which allow water flow to pass from one side of the sheet to the other, thereby reducing the resistance to waves and currents and enabling a flow of nutrients in the water to circulate through the carrier,
- wherein each carrier (11) is floating in the water by means of its positive buoyancy, and/or by being provided with buoyant means (40,28), and
(ii) suspending and arranging of said carrier (11) in the sea by attachment to sea bottom moored means for attachment (19,38,39,41,42), at one point or edge of the carrier sheet.

18. Method according to claim 17, wherein the means of attachment are moored by single point mooring to the sea bottom.

19. Method according to claim 18, wherein the seeding or provision of spores to the carrier is a method selected from:
i) spraying or other application onshore;
ii) spraying offshore in the sea; and
iii) natural spore attachment.

20. Method according to claim 19, wherein the seeding is a method selected from spraying with a nozzle, immersing in a bath, rolling with fluid suspension, strewing and electrostatical means.

## Patentansprüche

1. Am Meeresboden festgemachte Trägeranordnung zur automatisierten Aussaat, zum Züchten und Ernten von Makroalgen im Meer, die einen oder mehrere im Wasser aufgehängte Träger (11), die geeignet sind, wechselnden Fließrichtungen standzuhalten, und Einrichtungen zur Befestigung des Trägers am Festmacher aufweist, wobei
- jeder Träger (11) eine Platte aus einem biegsamen Material aufweist, das aus der Gruppe ausgewählt wird, die aus einem festen, einem mehrschichtigen und einem verstärkten Material besteht, wobei die Oberfläche der Platte (11) konfiguriert ist, Samen und Sporen zurückzuhalten,
- die Platte Öffnungen (12) hat, die den Durchgang eines Wasserstroms von einer Seite der Platte zur anderen erlauben, wodurch der Widerstand gegen Wellen und Strömungen verringert und die Zirkulation eines Stroms von Nährstoffen im Wasser durch den Träger hindurch ermöglicht wird,
- jeder Träger (11) mittels seines positiven Auftriebs und/oder durch Ausstattung mit schwimmfähigen Einrichtungen (40, 28) im Wasser treibt, und
- der Träger (11) mit den Befestigungseinrichtungen (19, 38, 39, 41, 42) an einem Punkt oder einer Kante der Platte verbunden ist.

2. Anordnung nach Anspruch 1, wobei der Träger oder die Gruppe von Trägern durch Einzelpunktfestmachung mittels einer Festmacheleine (26, 36, 39) am Meeresboden festgemacht wird.

3. Anordnung nach einem der vorhergehenden Ansprüche, wobei die Oberfläche der Platte rau, zerfurcht oder porös ist, um Saatgut und Sporen zurückzuhalten.

4. Anordnung nach Anspruch 1, **dadurch gekennzeichnet, dass** die Öffnungen (12) allgemein parallele Schlitze oder Reihen von allgemein kreisförmigen Öffnungen sind.

5. Anordnung nach einem der Ansprüche 1 bis 4, **dadurch gekennzeichnet, dass** der Träger rechtwinklig, blattförmig oder länglich ist, mit einem Rand (14, 15) auf mindestens einer Seite zum Aufhängen.

6. Anordnung nach Anspruch 5, **dadurch gekennzeichnet, dass** der Rand (14, 15) eine eingebettete Leine (16, 17) zur Verstärkung und zum Aufhängen hat.

7. Anordnung nach einem der vorhergehenden Ansprüche, **dadurch gekennzeichnet, dass** sie eine Schichtstruktur mit einem relativ starken und dünnen Folienmaterial und einer Lage von Wuchsstoff hat, der biologisch abbaubar sein kann.

8. Anordnung nach einem der Ansprüche 1 bis 7, **dadurch gekennzeichnet, dass** die Platte ein Netz von Verstärkungsleinen enthält.

9. Anordnung nach einem der Ansprüche 1 bis 8, **dadurch gekennzeichnet, dass** sie ein Düngemittel und/oder eine Nährsubstanz zur Verstärkung des Wachstums der Makroalgen aufweist.

10. Anordnung nach einem der Ansprüche 1 bis 9, **dadurch gekennzeichnet, dass** der Auftrieb entlang der Länge eines ausgestreckten Trägers variiert, um eine Wellenform zu erhalten, wenn er im Wasser aufgehängt ist.

11. Anordnung nach Anspruch 1, insbesondere mit einem rechtwinkligen Träger, **dadurch gekennzeichnet, dass** eine biegsame Stange (42) mit Auftrieb an einer Seite des Trägers befestigt oder in sie integriert ist, und eine steifere Stange (43) an einer benachbarten Seite befestigt ist, wobei die steifere Stange zum Aufhängen des Trägers (41) in einer allgemein senkrechten Stellung mit der Freiheit, dem Fließen des Meeres zu folgen, vorgesehen ist.

12. Anordnung nach Anspruch 1, **dadurch gekennzeichnet, dass** sie Befestigungseinrichtungen zum Aufhängen eines Trägers in Form eines allgemein kreisförmigen Elements (48) mit Auftrieb und mit Leinen oder einem Netz oder einer Folie oder Gewebe aufweist, die die Kante nach Art eines Fallschirms verbinden, wobei die Leinen und/oder das Netz mit einem Gewicht (51) verbunden sind, und wobei das kreisförmige Element (48) eine Plattform und der Träger darauf angeordnet ist.

13. Anordnung nach Anspruch 1, wobei eine Gruppe von Trägern an einer Befestigungseinrichtung wie einer Leine (19), einem Stamm (38, 39) oder zentralem Ring (42) befestigt ist.

14. Verwendung einer am Meeresboden festgemachten Trägeranordnung zur automatisierten Aussaat, zum Züchten und Ernten von Seetang, wobei die Anordnung einen oder mehrere im Wasser aufgehängte Träger (11), die geeignet sind, wechselnden Fließrichtungen standzuhalten, und Einrichtungen zur Befestigung des Trägers am Festmacher aufweist, wobei
- jeder Träger (11) eine Platte aus einem biegsamen Material aufweist, das aus der Gruppe ausgewählt wird, die aus einem festen, einem mehrschichtigen und einem verstärkten Material besteht, wobei die Oberfläche der Platte (11) konfiguriert ist, Samen und Sporen zurückzuhalten,
- die Platte Öffnungen (12) hat, die den Durchgang eines Wasserstroms von einer Seite der Platte zur anderen erlauben, wodurch der Widerstand gegen Wellen und Strömungen verringert und die Zirkulation eines Stroms von Nährstoffen im Wasser durch den Träger hindurch ermöglicht wird,
- jeder Träger (11) mittels seines positiven Auftriebs und/oder durch Ausstattung mit schwimmfähigen Einrichtungen (40, 28) im Wasser treibt, und
- der Träger (11) mit den Befestigungseinrichtungen (19, 38, 39, 41, 42) an einem Punkt oder einer Kante der Platte verbunden ist.

15. Verwendung nach Anspruch 14, wobei der Träger senkrecht oder waagrecht im Wasservolumen aufgehängt ist.

16. Verwendung nach Anspruch 14, wobei der Träger aus Kunststoff, organischem Material oder einer Kombination hergestellt ist, die eine große Anzahl von Sporen oder Samen für den Wuchs halten und ein durchgehendes Aufgehängtsein in Wasser aushalten kann.

17. Verfahren zum Züchten von Seetang, **dadurch gekennzeichnet, dass** das Verfahren die folgenden Schritte aufweist:
(i) Säen der Samen oder Sporen auf mindestens einen Träger zum Züchten von Seetang, wobei der Träger eine Platte aus einem biegsamen Material aufweist, das aus der Gruppe ausgewählt wird, die aus einem festen, einem mehrschichtigen und einem verstärkten Material ausgewählt wird, wobei die Oberfläche der Platte (11) konfiguriert ist, Samen und Sporen zurückzuhalten, die darauf abgelagert wurden,
- wobei die Platte Öffnungen (12) hat, die den Durchgang eines Wasserstroms von einer Seite der Platte zur anderen erlauben, wodurch der Widerstand gegen Wellen und Strömungen verringert und die Zirkulation eines Stroms von Nährstoffen im Wasser durch den Träger hindurch ermöglicht wird,
- wobei jeder Träger (11) mittels seines positiven Auftriebs und/oder durch Ausstattung mit schwimmfähigen Einrichtungen (40, 28) im Wasser treibt, und
(ii) Aufhängen und Anordnen des Trägers (11) im Meer durch Befestigung an am Meeresboden festgemachten Befestigungseinrichtungen (19, 38, 39, 41, 42) an einem Punkt oder einer Kante der Trägerplatte.

18. Verfahren nach Anspruch 17, wobei die Befestigungseinrichtungen durch Einzelpunktfestmachung am Meeresboden festgemacht werden.

19. Verfahren nach Anspruch 18, wobei die Aussaat oder das Liefern von Sporen an den Träger ein Verfahren ist, das ausgewählt wird aus:
i) Sprühen oder anderes Auftragen an Land;
ii) Sprühen auf offener See im Meer; und
iii) natürliche Sporenhaftung.

20. Verfahren nach Anspruch 19, wobei die Aussaat ein Verfahren ist, das ausgewählt wird aus Sprühen mit einer Düse, Eintauchen in ein Bad, Einwalzen mit einer flüssigen Suspension, Aufstreuen und elektrostatische Mittel.

## Revendications

1. Disposition porteur ancré au fond de la mer pour lesemis, la culture et la récolte automatisés des macro-algues dans la mer, composée d'un ou plusieurs porteurs (11) suspendus dans l'eau, adaptés à supporter des directions de flux variables, et un moyen de fixer le porteur à un ancrage, **caractérisée en ce que**
- chaque porteur (11) comporte une feuille de matériau flexible choisi dans le groupe composé d'un matériau solide, d'un matériau lamellé et d'un matériau renforcé, où la surface de la feuille (11) est configurée de manière à retenir les graines et les spores,
- la feuille présente des ouvertures (12) qui permettent à un flux d'eau de passer d'un côté de la feuille à l'autre, réduisant ainsi la résistance aux vagues et aux courants et permettant au flux de nutriments dans l'eau de circuler à travers le porteur,
- où chaque porteur (11) flotte dans l'eau au moyen de sa flottabilité positive, et/ou en étant équipé de moyen de flottaison (40, 28) et
- le porteur (11) étant connecté au moyen de fixation (19, 38, 39, 41, 42) au niveau d'une pointe ou d'un bord de la feuille.

2. Disposition selon la revendication 1, **caractérisée en ce que** le porteur ou groupe de porteurs est ancré au fond de la mer par un ancrage à point unique via une amarre (26, 36, 39).

3. Disposition selon l'une quelconque des revendications précédentes, **caractérisée en ce que** la surface de la feuille est rugueuse, accidentée ou poreuse afin de retenir les graines et les spores.

4. Disposition selon la revendication 1, **caractérisée en ce que** les ouvertures (12) sont généralement des fentes parallèles ou des séries d'ouvertures généralement circulaires.

5. Disposition selon l'une des revendications 1 à 4, **caractérisée en ce que** le porteur est rectangulaire, en forme de feuille ou oblong, avec une bordure (14, 15) sur au moins un côté pour la suspension.

6. Disposition selon la revendication 5, **caractérisée en ce que** la bordure (14, 15) est pourvue d'un câble intégré (16, 17) à des fins de renforcement et de suspension.

7. Disposition selon l'une quelconque des revendications précédentes, **caractérisée en ce qu'**il comprend une structure lamellée dans un matériau relativement résistant et en feuille fine et une couche de substance de croissance, qui peut être biodégradable.

8. Disposition selon l'une quelconque des revendications 1 à 7, **caractérisée en ce que** la feuille inclut un réseau de câbles de renforcement.

9. Disposition selon l'une quelconque des revendications 1 à 8, **caractérisée en ce qu'**elle comprend un fertilisant et/ou une substance nutritive afin d'améliorer la croissance des macro-algues.

10. Disposition selon l'une des revendications 1 à 9, **caractérisée en ce que** la flottabilité est variable le long de la longueur d'un porteur allongé, afin qu'il adopte une forme ondulée quand il est en suspension dans l'eau.

11. Disposition selon la revendication 1, en particulier avec un porteur de forme rectangulaire, **caractérisée en ce qu'**une tige flexible (42) flottante est fixée ou intégrée au niveau d'un côté du porteur, et une tige plus rigide (43) est fixée au niveau d'un côté adjacent, la dite tige plus rigide étant prévue pour la suspension du porteur (41) dans une position généralement verticale, avec la liberté de suivre le courant de la mer.

12. Disposition selon la revendication 1, **caractérisée en ce qu'**elle comprend un moyen de fixation pour suspendre un porteur sous la forme d'un élément généralement circulaire (48) flottant et avec des câbles ou un filet ou une feuille ou un tissu connectant le bord à la façon d'un parachute, les dits câbles et/ou filet étant connectés à un poids (51) et où l'élément circulaire (48) est une plateforme et le dit porteur est placé dessus.

13. Disposition selon la revendication 1, **caractérisée en ce qu'**un groupe de porteurs est fixé à un moyen de fixation tel qu'un câble (19), une tige (38, 39) ou anneau central (42).

14. Utilisation d'une disposition de porteurs ancrés au fond de la mer pour le semis, la culture et la récolte automatiques des algues, **caractérisée en ce que** la dite disposition comprend un ou plusieurs porteurs (11) suspendus dans l'eau, adaptés à supporter des flux d'eau variables et un moyen de fixer le porteur à l'ancrage, où
- chaque porteur (11) comprend une feuille de matériau flexible choisi dans le groupe composé d'un matériau solide, un matériau lamellé et un matériau renforcé, où la surface de la feuille (11) est configurée de manière à retenir les graines et les spores,
- la feuille ayant des ouvertures (12) qui permettent à l'eau de passer d'un côté de la feuille à l'autre, réduisant ainsi la résistance aux vagues et aux courants et permettant à un flux de nutriments dans l'eau de circuler à travers le porteur,
- où chaque porteur (11) flotte dans l'eau grâce à sa flottabilité positive, et/ou en étant équipé de moyen de flottaison (40, 28), et
- le porteur (11) étant connecté au moyen de fixation (19, 38, 39, 41, 42) au niveau d'une pointe ou d'un bord de la feuille.

15. Utilisation selon la revendication 14, **caractérisée en ce que** le porteur est suspendu verticalement ou horizontalement dans le volume d'eau.

16. Utilisation selon la revendication 14, **caractérisée en ce que** le porteur est fabriqué en matière plastique, organique ou une combinaison, qui peut retenir un grand nombre de spores ou de graines pour la croissance et qui peut supporter d'être continuellement en suspension dans l'eau.

17. Méthode de culture des algues, **caractérisée en ce que** la méthode comprend les étapes suivantes :
(i) semer les graines ou spores sur au moins un porteur pour faire pousser les algues, où le dit porteur comprend une feuille de matériau flexible choisi dans le groupe composé d'un matériau solide, lamellé et renforcé, où la surface de la feuille (11) est configurée pour retenir les graines et les spores déposées dessus,
- la feuille ayant des ouvertures (12) qui permettent à l'eau de passer d'un côté de la feuille à l'autre, réduisant ainsi la résistance aux vagues et aux courants et permettant à un flux de nutriments dans l'eau de circuler à travers le porteur,
- où chaque porteur (11) flotte dans l'eau grâce à sa flottabilité positive et/ou en étant équipé de moyens de flottaison (40, 28), et
(ii) mettre le dit porteur (11) en suspension et le placer dans la mer en le fixant au moyen d'ancrage au fond de la mer (19, 38, 39, 41, 42) au niveau d'une pointe ou d'un bord de la feuille du porteur.

18. Méthode selon la revendication 17, **caractérisée en ce que** le moyen de fixation est ancré par un ancrage unique au fond de la mer.

19. Méthode selon la revendication 18, **caractérisée en ce que** le semis ou l'apport de spores au porteur est une méthode choisie parmi :
i) la pulvérisation ou une autre application terrestre,
ii) la pulvérisation au large, et
iii) la fixation naturelle des spores.

20. Méthode selon la revendication 19, **caractérisée en ce que** le semis est une méthode choisie entre la pulvérisation avec un jet, l'immersion dans un bain, l'enroulement dans une suspension de fluide, la dispersion et un moyen électrostatique.
